# EUROPÄISCHE PATENTSCHRIFT

(11) **EP 3 228 299 B1**
(45) Veröffentlichungstag und Bekanntmachung des Hinweises auf die Patenterteilung: **24.06.2020**
(21) Anmeldenummer: 17159514.3
(22) Anmeldetag: 07.03.2017
(51) Int. Cl.: A61Q 17/04, A61K 8/35, A61K 8/49

(54) **NEUES SONNENSCHUTZMITTEL MIT REDUZIERTER NEIGUNG ZUR TEXTILVERFLECKUNG**
NOVEL SUNSCREEN COMPOSITION WITH A REDUCED TEXTILE STAINING
PROTECTION SOLAIRE AYANT UNE TENDANCE RÉDUITE À TÂCHER LES TISSUS

(30) Priorität: 05.04.2016 DE 102016205580
(43) Veröffentlichungstag der Anmeldung: 11.10.2017
(73) Patentinhaber: Beiersdorf AG, 20253 Hamburg (DE)
(72) Erfinder: Sprock, Sarah, 22297 Hamburg (DE); Eisert, Anja, 22085 Hamburg (DE); Göddertz, Dominik, 22763 Hamburg (DE)

(56) Entgegenhaltungen:
- DE-A1-102010 008 320
- DE-A1-102014 104 256
- DE-A1-102014 207 935

## Beschreibung

Die vorliegende Erfindung betrifft Verfahren und Verwendungen zur Erleichterung der Auswaschbarkeit von UV-Lichtschutzfilter enthaltenden kosmetischen Zubereitungen aus Textilien, dadurch gekennzeichnet, dass dem Kosmetikum 4-Hydroxyacetophenon zugesetzt wird.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und Wege zu finden, um eine kosmetische Zubereitung (insbesondere ein Sonnenschutzmittel) enthaltend nicht-wasserlösliche UV-A-Rlter und/oder Breitbandfilter leichter aus den mit der Zubereitung kontaminierten Textilen herauswaschen zu können.

Überraschend gelöst wird die Aufgabe durch ein Verfahren zur Erleichterung der Auswaschbarkeit von UV-Uchtschutzfilter enthaltenden kosmetischen Zubereitungen aus Textilien, wobei die kosmetische Zubereitung 4-(tert.-Butyl)-4'-methoxydibenzoylmethan (INCI Butyl Methoxydibenzoylmethane) und/oder 2,4-Bis-{[4-(2-ethyl-hexyloxy)-2-hydroxy]-phenyl}-6-(4-methoxyphenyl)-1,3,5-triazin (INCI Bis-Ethythexyloxyphenol Methoxyphenyl Triazine) enthält, dadurch gekennzeichnet, dass dem Kosmetikum, bezogen auf das Gesamtgewicht der Zubereitung, 0,01 bis 2 Gewichts-% 4-Hydroxyacetophenon zugesetzt wird.

Überraschend gelöst wird die Aufgabe durch ein Verfahren zur Reduzierung der durch UV-Uchtschutzfilter enthaltenden kosmetischen Zubereitungen hervorgerufenen Textilverfleckung, wobei die kosmetische Zubereitung 4-(tert.-Butyl)-4'-methoxydibenzoylmethan (INCI Butyl Methoxydibenzoylmethane) und/oder 2,4-Bis-([4-(2-ethyl-hoxyloxy)-2-hydroxy]-phonyl)-6-(4-methoxyphenyl)-1,3,5-triazin (INCI Bis-Ethylhexyloxyphenol Methoxyphenyl Triazine) enthält, dadurch gekennzeichnet, dass dem Kosmetikum, bezogen auf das Gesamtgewicht der Zubereitung, 0,01 bis 2 Gewichts-% 4-Hydroxyacetophenon zugesetzt wird.

Überraschend gelöst wird die Aufgabe durch die Verwendung von 4-Hydroxyacetophenon in UV-Lichtschutzfilter enthaltenen kosmetischen Zubereitungen zur Erleichterung der Auswaschbarkeit der UV-Lichtschutzfilter aus mit den Zubereitungen kontaminierten Textilien, wobei die kosmetische Zubereitung 4-tert.-Butyl)-4'-methoxydibenzolmethan (INCI Butyl Methoxydibenzoylmethane) und/oder 2,4-Bis-{[4-(2-ethyl-hexyloxy)-2-hydroxy]-phenyl)-6-(4-methoxyphenyl)-1,3,5-triazin (INCI Bis-Ethylhexyloxyphenol Methoxyphenyl Triazine) enthält und die kosmetische Zubereitung bzw. das Kösmetikum von 0,01 bis 2 Gewichts-% 4-Hydroxyacatophenon, bezogen auf das Gesamtgewicht der Zubereitung, enthält.

Überraschend gelöst wird die Aufgabe durch die Verwendung von 4-Hydroxyacetophenon in UV-Lichtschutzfilter enthaltenden kosmetischen Zubereitungen zur Reduzierung der durch die Zubereitung hervorgerufende Textilverfleckung, wobei die kosmetische Zubereitung 4-(tert.-Butyl)-4'-methoxydibenzoylmethan (INCI Butyl Methoxydibenzoylmethane) und/oder 2,4-Bis{[4-(2-ethyl-hexyloxy)-2-hydroxy]-phenyl]-6-(4-methoxyphenyl)-1,3,5-triazin (INCI Bis-Ethylhexyloxyphenol Methoxyphenyl Triazine) enthält und die kosmetische Zubereitung bzw. das Kosmetikum von 0,01 bis 2 Gewichts-% 4-Hydroxyacetophenon, bezogen auf das Gesamtgewicht der Zubereitung, enthält.

Zwar kennt der Fachmann die WO2011/101250, DE 10 2014 104255, DE 10 2014 104256 sowie die EP 2939710, das heißt, dem Fachmann waren Sonnenschutzmittel mit 4-Hydroxyacetophenon an sich bekannt. Dennoch konnten diese Schriften nicht den Weg zur vorliegenden Erfindung weisen, da die Wirkung des 4-Hydroxyacetophenons auf die Auswaschbarkeit nicht erkannt wurde. Diese Wirkung war umso überraschender, da 4-Hydroxyacetophenon an sich nicht besonders gut wasserlöslich ist.
Darüber hinaus kennt der Fachmann die DE 10 2010 008320 und DE 10 2014 207935, die ebenfalls nicht den Weg zur vorliegenden Erfindung weisen konnten.

4-Hydroxyacetophenon, auch als p-Hydroxyacetophenon oder 1-(4-Hydroxyphenyl)-ethanon bezeichnet (INCI: Hydroxyacetophenon), kann beispielsweise bei der Firma Symrise unter dem Handelsnamen Symsave H erwogen werden.

Die Begriffe "erffindungsgemaße Zubereitung", "erfindungsgemäß" etc. beziehen sich im Rahmen der vorliegenden Offenbarung immer auf das erfindungsgemäße Verfahren und die erfindungsgemäße Verwendung.

Die Begriffe kosmetische Zubereitung und Kosmetikum werden synonym verwendet.

Es ist. erfindungsgemäß, wenn die kosmetische Zubereitung bzw. das Kosmetikum die Verbindungen 4-(tert-Butyl)-4'-methoxydibenzoylmethan (INCI Butyl Methoxydibenzoylmethane) und/oder 2,4-Bis-{[4-(2-ethyl-hexyloxy)-2-hydroxy]-phenyl]-6-(4-methoxyphenyl)-1,3,5-triazin (INCI Bis-Ethylhexytoxyphenol Methoxyphenyl Triazine) enthält.

Dabei sind die drei erfindungsgemäßen Varianten wie folgt gekennzeichnet:
Enthält die kosmetische Zubereitung 4-(tert.-Butyl)-4-methoxydibezoylmethan (INCI Butyl Methoxydibenzoylmethane) und kein Bis-Ethylhexyloxyphenol Methoxyphenyl Triazine, so ist es erfindungsgemäß vorteilhaft, wenn der Gehalt an 4-(tert-Butyl)-4'-methoxydiben-zoylmethan (INCI Butyl Methoxydibenzoylmethane) von 0,1 bis 5 Gewichts-%, bezogen auf das Gesamtgewicht der Zubereitung beträgt.

Enthält die kosmetische Zubereitung 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) und kein Butyl Methoxydibenzoylmethane, so ist es erfindungsgemäß vorteilhaft, wenn der Gehalt an 2,4-Bis-{[4-(2-ethyl-hexiloxy)-2-hydroxy]-phenyl)-6-(4-methoxyphenyl)-1,3,5-triazin (INCI Bis-Ethylhexyloxyphenol Methoxyphenyl Triazine) von 0,1 bis 10 Gewichts-%. bezogen auf das Gesamtgewicht der Zubereitung beträgt.

Enthält die kosmetische Zubereitung 4-(tert.-Butyl)-4'-methoxydibenzoylmethan (INCI Butyl Methoxydibenzoylmethane) und 2,4-Bis-{[4-(2-ethyl-hexyloxy)-2-hydroxy)-phenyl}-6-(4-methoxyphenyl)-1,3,5-triazin (INCI Bis-Ethylhexyloxyphenol Methoxyphenyl Triazine), so ist es erfindungsgemäß vorteilhaft, wenn der Gehalt an 4-(tert-Butyl)-4'-methoxydiben-zoylmethan (INCI Butyl Methoxydibenzoylmethane) von 0,1 bis 5 Gewichts-% und der Gehalt an 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) von 0,1 bis 10 Gewichts-% jeweils bezogen auf das Gesamtgewicht der Zubereitung beträgt.

Es ist für das erfindungsgemäße Verfahren und die erfindüngsgemäße Verwendung erfindungsgemäß bevorzugt wenn die kosmetische Zubereitung bzw. das Kosmetikum von 0,1 bis 1 Gewichts-% 4-Hydroxyacetophenon, bezogen auf das Gesamtgewicht der Zubereitung, enthält

Darüber hinaus kann die kosmetische Zubereitung vorteilhaft noch weitere UV-Filter enthalten, die beispielsweise gewählt werden aus der Gruppe der Verbindungen 2-Phenylbenzimidazol-5-sulfonsäure und/oder deren Salze; Phenylen-1,4-bis-(2-benzimidazyl)-3,3'-5,5-tetrasulfonsäuresalze; 1,4-di(2-oxo-10-Sulfo-3-bornylidenmethyl)-Benzol und dessen Salze; 4-(2-Oxo-3-bornylidenmathyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-etramethyl-1-[(trimethylsilyl)oxy]disiloxanyl]propyl]-phenol; 3-(4-Methylbenzyliden)campher, 3-Benzylidencampher; Ethylhexylsalicylat; Terephthalidendicamphersulfonsäure; 2-Ethylhexyl-2-cyano-3,3-diphenylacrylat; 4-(Dimethyamino)-benzoasäure(2-ethylhexyl)ester; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-mathylbenzophenon; 2,2-Dihydoxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbanylvinyl)-phenoxy)propenyl-methoxysiloxan / Dimethylslioxan - Copolymer; Dioctylbutylamidotriazon (INCI: Diethylhexyl-Butamidotriazone); 2,4-bis-[5-1(dimethylpropyl)benzoaxazol-2-yl-(4-phenyl)-imino]-6-(2-ethylhexyl)-mino-13,6-triazin mit der (CAS Nr. 288254-16-0); 4,4',4"-(13,5'Triazin-2,4,6-triyltriimino)-tris-benzoasäüre-tris(2-ethylhexylester) (auch; 2,4,6-Tris-[anilino-(p-carbo-2'-ethyl-1'-hexyloxy)]-1,3,5-triazin (INCI: Ethylhexyl Triazone); 2,4,6-Trihiphenyl-yl-1,3,5-triazin; Merocyanine; Piperazinderivate;Titandioxid; Zinkoxid.

Dabei ist es erfindungsgemäß von Vorteil, wenn die erfindungsgemäße Zubereitung frei ist von 2-Ethylhexyl-2-cyano-3,3-diphenylacrilat, 2-Hydroxy-4-methoxybenzophenon und/oder 3-(4-Methylbenzyliden)campher. Dabei sollte bevorzugt auf den Einsatz von 3-(4-Methylbenzyliden)campher und 2-Hydroxy-4-methoxybenzophenon verzichtet werden.

Der erfindungsgemäße Effekt kann dadurch erfindungsgemäß verbessert werden, dass man dem Kosmetikum ein oder mehrere Komplexbildner zusetzt.

Dabei ist es erfindungsgemäß bevorzugt, wenn als Komplexbildner eine oder mehrere der Verbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat (IDS)
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA) und/oder deren Alkalisalze eingesetzt werden.

Es ist erfindungsgemäß besonders bevorzugt, wenn als Komplexbildner eine oder mehrere der Verbindungen aus der Gruppe
- Diethylentriaminpenta(methylenphosphonsäure)/ DTPMP
   - Ethylendiamintetra(methylenphosphonsäure/ EDTMP
   - Aminotrimethylenphosphonsäure/ ATMP
   - Phosphonobutan-tricarbonsäure/ PBTC
   - Iminodisuccinat (IDS)
   - Natriumpolyphosphat
   - Tetranatriumpyrophosphat
   - Bernsteinsäure
      und/oder deren Alkalisalze und/ oder deren Amin-N-Oxide eingesetzt werden.

Die erfindungsgemäß bevorzugte Gesamt-Einsatzkonzentration für die Komplexbildner (einer oder mehrere) beträgt dabei von 0,01 bis 3 Gewichts-%, bezogen auf das Gesamtgewicht der Zubereitung.

Der erfindungsgemäße Effekt kann dadurch erfindungsgemäß verbessert werden, dass man dem Kosmetikum 1-Hydroxy-4-methyl-6-(2,4,4-trimethylpentyl)-2(1H)-pyridon (Pirocton) und/oder dessen Monoethanolamin-Salz zugesetzt wird.

Die erfindungsgemäß bevorzugte Gesamt-Einsatzkonzentration für Pirocton beträgt dabei von 0,01 bis 1 Gewichts-%, bezogen auf das Gesamtgewicht der Zubereitung.

Der erfindungsgemäße Effekt kann dadurch erfindungsgemäß verbessert werden, dass man dem Kosmetikum Siloxanelastomere zusetzt.

Die erfindungsgemäß bevorzugte Gesamt-Einsatzkonzentration für Siloxanelastomere (einer oder mehrere) beträgt dabei von 0,1 bis 20 Gewichts-%, bezogen auf das Gesamtgewicht der Zubereitung.

Nicht zuletzt kann der erfindungsgemäße Effekt dadurch erfindungsgemäß verbessert werden, dass man dem Kosmetikum ein oder mehrere Polysaccharide zusetzt.

Die erfindungsgemäßen Polysaccharide können aus unterschiedlichen Stoffgruppen gewählt werden.

So ist eine erfindungsgemäß vorteilhafte Ausführungsform der vorliegenden Erfindung dadurch gekennzeichnet, dass die verwendeten Polysaccharide ausgewählt werden aus der Gruppe der Gumen.

In einem solchen Fall ist es erfindungsgemäß bevorzugt, wenn die verwendeten Polysaccharide ausgewählt werden aus der Gruppe der Verbindungen Welan Gum, Sclerotium Gum und Cellulose Gum.

Die erfindungsgemäß vorteilhaften Polysaccharide können jedoch auch ausgewählt werden aus der Gruppe der Verbindungen Alginate (insbesondere Sodium Alginate) und Carboxymethylcellulose.

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Für die erfindungsgemäß vorteilhaften Polysaccharide gelten für die einzelnen Stoffe die folgenden Einsatzkonzentrationen als erfindungsgemäß bevorzugt:
Welan Gum wird erfindungsgemäß bevorzugt in einer Konzentration von 0,01 bis 1,5 Gewichts-%, bezogen auf das Gesamtgewicht der Zusammensetzung eingesetzt. Als Welan Gum kann beispielsweise der Rohstoff Collstab W-100 der Firma Colltec vorteilhaft eingesetzt werden.

Sclerotium Gum wird erfindungsgemäß bevorzugt in einer Konzentration von 0,01 bis 1,5 Gewichts-%, bezogen auf das Gesamtgewicht der Zusammensetzung eingesetzt. Als Sclerotium Gum kann beispielsweise der Rohstoff Actigum CS 11 der Firma Cargill vorteilhaft eingesetzt werden.

Cellulose Gum wird erfindungsgemäß bevorzugt in einer Konzentration von 0,01 bis 1,5 Gewichts-%, bezogen auf das Gesamtgewicht der Zusammensetzung eingesetzt. Als Cellulose Gum kann beispielsweise der Rohstoff Blanose Cellulose Gum der Firma Ashland vorteilhaft eingesetzt werden.

Sodium Alginate wird erfindungsgemäß bevorzugt in einer Konzentration von 0,01 bis 1,5 Gewichts-%, bezogen auf das Gesamtgewicht der Zusammensetzung eingesetzt. Als Sodium Alginate kann beispielsweise der Rohstoff Alginic Acid Sodium Salt der Firma Sigma Aldrich vorteilhaft eingesetzt werden.

Carboxymethylcellulose wird erfindungsgemäß bevorzugt in einer Konzentration von 0,01 bis 2 Gewichts-%, bezogen auf das Gesamtgewicht der Zusammensetzung eingesetzt. Als Carboxymethylcellulose kann beispielsweise der Rohstoff Aqualon CMC der Firma Ashland vorteilhaft eingesetzt werden.

Erfindungsgemäß vorteilhafte Ausführungsformen der vorliegenden Erfindung sind dadurch gekennzeichnet, dass die Zubereitung in Form einer Öl-in -Wasser-Emulsion (O/W-Emulsion) vorliegt.
In einem solchen Fall sind die erfindungsgemäß bevorzugten Ausführungsformen dadurch gekennzeichnet, dass die Zubereitung einen oder mehrere Emulgatoren gewählt aus der Gruppe der Verbindungen Glycerylstearatcitrat, Cetearylalkohol Natriumcetearylsulfat + Glycerylstearat, Cetearylsulfosuccinat Natriumstearoylglutamat, Polyglyceryl-3-methylglucosedistearat, Stearinsäure, Kaliumcetylphosphat, enthalten.

Darüber hinaus kann die kosmetische Zubereitung wie ein für solche Fälle übliches Kosmetikum zusammengesetzt sein und die entsprechenden, bekannten Inhaltsstoffe enthalten.

### Vergleichsversuch/Ausführungsbeispiel

Waschversuch mit dem LINI-Test von folgenden Formeln:
Formel A (O/W Sonnenschutzlotion SPF 30 ohne Hydroxyacetophenone)
Formel B (Formel A + 0,6% Hydroxyacetophenone
Formel C (O/W Sonnenschutzlotion SPF 50+ ohne Hydroxyacetophenone)
Formel D (Formel B + 1% Hydroxyacetophenone)

Alle Formeln enthalten die Verbindungen 4-(tert.-Butyl)-4'-methoxydibenzoylmethan (INCI Butyl Methoxydibenzoylmethane) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine).

### Fleckenmonitor:

Trockene, vorgewaschene Baumwolllappen
1) Die Versuchsformeln werden mithilfe von PMMA-Platten auf die Fleckenmonitore appliziert (2mg/ cm²). Pro Formel werden vier Flecken erzeugt.
   Die Flecken werden über Nacht trocknen gelassen.
2) Mithilfe eines Spektro-Pens (Dr. Lange spectro-pen) werden die l*- ,a*- und b*-Werte sowohl des nicht befleckten Baumwolllappens als auch die der getrockneten Flecken aufgenommen (10 Messpunkte je Fleck).
3) Die Flecken werden in einem LINI-Test mit Stahlkügelchen als Beiladung gewaschen (60min bei 60°C). Die Waschlauge enthält 3,33g Pulver-Vollwaschmittel/L.
   Anschließend werden die Lappen 15min lang unter Rühren in Leitungswasser gespült.
4) Nachdem sie über Nacht getrocknet sind, werden wie unter 1) die l*- ,a*- und b*-Werte gemessen und die Verfleckung (insbesondere der b*Wert) vor, bzw. nach dem Waschvorgang berechnet.

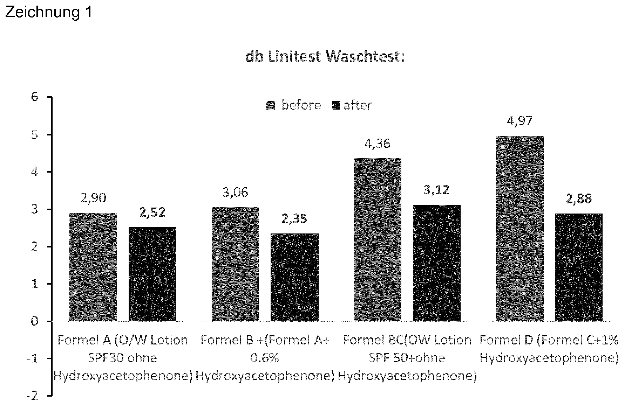

Es ergeben sich die in Zeichnung 1 abgebildeten Ergebnisse für die getesteten Formeln:
Links in der Zeichnung sind jeweils die Gelbwerte für das mit Sonnenschutzmittel präparierte Textil vor dem Waschen und rechts nach dem Waschen angegeben.

Fazit: Es zeigte sich, dass durch das erfindungsgemäße Verfahren bzw. die erfindungsgemäße Verwendung sich die UV-Filter 4-(tert.-Butyl)-4'-methoxydibenzoylmethan (INCI Butyl Methoxydibenzoylmethane) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) leichter und vollständiger aus dem Baumwollträger auswaschen lassen und die durch diese UV-Filter hervorgerufene Textilverfleckung signifikant reduziert wird.

### Beispiele für Rezepturen/Sonnenschutzmittel mit denen das erfindungsgemäße Verfahren und die erfindungsgemäße Verwendung durchgeführt werden können:

Alle Mengenangaben, Anteile und Prozentanteile sind, soweit nicht anders angegeben, auf das Gewicht und die Gesamtmenge bzw. auf das Gesamtgewicht der Zubereitungen bezogen.

| **1) O/W Sonnenschutzlotion** | **Gew.%** |
|---|---|
| Cetyl Alcohol | 1.0 |
| Glyceryl Stearate Citrate | 2.0 |
| C12-15 Alkyl Benzoate | 6.0 |
| Stearyl Alcohol | 0.5 |
| Homosalate | 8.5 |
| Octocrylene | 9.0 |
| Ethylhexyl Salicylate | 3.5 |
| Butyl Methoxydibenzoylmethane | 4.0 |
| Myristyl Myristate | 1.0 |
| Hydrogenated Coco-Glycerides | 1.0 |
| VP/Hexadecene Copolymer | 0.5 |
| Tocopheryl Acetate | 0.2 |
| Titanium Dioxide (nano) | 3.0 |
| Sodium Carboxymethylcellulose | 0.1 |
| Acrylates/C10-30 Alkyl Acrylate Crosspolymer | 0.1 |
| Xanthan Gum | 0.3 |
| Trisodium EDTA | 0.7 |
| Glycerin | 5.0 |
| Tetrasodium Iminodisuccinate | 0.5 |
| Sodium Hydroxide | q.s |
| Alcohol Denat. | 4.0 |
| Hydroxyacetophenone | 0.5 |
| Panthenol | 1.0 |
| Parfum | 0.4 |
| Ethylhexylglycerin | 0.3 |
| Aqua | add to 100 |

| **2) O/W Sonnenschutzlotion** | **Gew.%** |
|---|---|
| Sodium Carboxymethylcellulose | 0.5 |
| Tocopheryl Acetate | 0.06 |
| Hydroxyacetophenone | 0.6 |
| Ethylhexylglycerin | 0.5 |
| C12-15 Alkyl Benzoate | 3.0 |
| Butylene Glycol Dicaprylate/Dicaprate | 1.0 |
| C18-38 Alkyl Hydroxystearoyl Stearate | 1.5 |
| Glyceryl Stearate | 1.0 |
| Sodium Stearoyl Glutamate | 0.3 |
| Silica Dimethyl Silylate | 0.5 |
| Triacontanyl PVP | 1.0 |
| Glycerin | 2.0 |
| Sodium Hydroxide | q.s (pH 7.3) |
| Stearyl Alcohol | 1.0 |
| Acrylates/C10-30 Alkyl Acrylate Crosspolymer | 0.15 |
| Xanthan Gum | 0.4 |
| Alcohol Denat. | 5.0 |
| Trisodium EDTA | 0.8 |
| Tetrasodium Iminodisuccinate | 0.75 |
| Homosalate | 8.5 |
| Ethylhexyl Salicylate | 4.5 |
| Bis-Ethylhexyloxyphenol Methoxyphenyl Triazine | 2.0 |
| Ethylhexyl Triazone | 1.0 |
| Butyl Methoxydibenzoylmethane | 3.5 |
| Phenylbenzimidazole Sulfonic Acid | 1.0 |
| Aqua | add to 100 |

| **3) O/W Sonnenschutzspray** | **Gew.%** |
|---|---|
| Tocopheryl Acetate | 0.1 |
| Hydroxyacetophenone | 0.4 |
| Panthenol | 1.4 |
| Copernicia Cerifera Cera | 0.5 |
| Glyceryl Stearate | 1.0 |
| Sodium Stearoyl Glutamate | 0.4 |
| VP/Hexadecene Copolymer | 0.5 |
| Glycerin | 4.0 |
| Citric Acid | q.s (pH 7.3) |
| Phenoxyethanol | 0.5 |
| Sodium Carboxymethylcellulose | 0.5 |
| Xanthan Gum | 0.1 |
| Microcrystalline Cellulose + Cellulose Gum | 0.9 |
| Alcohol Denat. | 4.0 |
| Trisodium EDTA | 0.7 |
| Tetrasodium Iminodisuccinate | 0.8 |
| Homosalate | 8.5 |
| Octocrylene | 5.0 |
| Ethylhexyl Salicylate | 4.5 |
| Butyl Methoxydibenzoylmethane | 3.0 |
| Phenylbenzimidazole Sulfonic Acid | 0.5 |
| Aqua | Add to 100 |

## Patentansprüche

1. Verfahren zurErleictrterung der Auswaschbarkeit von UV-Lichtschutzfilter enthaltenden kosmetischen Zubereitungen aus Textilien, wobei die kosmetische Zubereitung 4-(tert-Butyl)-4'methoxydibenzoylmethäh (INCI Butyl Methoxydibenzoylmethane) und/oder 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält, **dadurch gekennzeichnet, dass** dem Kosmetikum, bezogen auf das Gesamtgewicht der 0,01 bis 2 Gewichits-% 4-Hydroxyacetophenon zugesetzt wird.

2. Verfahren zur Reduzierung der durch UV-Lichitschufilter enthaltenden kosmetischen Zubereitungen hervorgerufenen Textilverfleckung, wobei die kosmetische Zubereitung 4-(tert.-Buty)-4'-methoxydibenzoylmethan (INCI Butyl Methoxydibenzoylmethane und/oder 2,4-bis-{[4-(2-ethyl-hexyloxy)-2-hydroxy]-phenyl}-6-(4-methoxyphenyl)-1,3,5-triazin (INCI Bis-Bhylhexyloxyphenol Methoxyphenyl Triazine) enthält, **dadurch gekennzeichnet, dass** dem Kosmetikum, bezogen auf das Gesamtgewicht der Zubereitung, 0,01 bis 2 Gewichts-% 4-Hydroxyacetophenen zugesetzt wird.

3. Verwendung von 4-Hydroxyacetophenon in UV-Lichtschutzfilter enthaltenden kosmetischen Zubereitungen zur Erleichterung der Auswaschbarkeit der UV-Lichtschützfilter aus mit den Zubereitungen kontaminierten Textilien, wobei die kosmetische Zubereitung 4-(tert.-Butyl)-4-methoxydibenzoylmethan (INCI Butyl Methoxydibenzoylmethane) und/oder 2,4-Bis-{[2-ethyl-hexyloxy)-2-hydroxy]-phenyl]-6-(4-methoxyphenyl)-1,3,5-triazin (INCI Bis-Ethylhexyloxyphenol Methoxyphenyl Triazine) enthält und die kosmetische Zubereitung bzw. das Kosmetikum von 0,01 bis 2 Gewichts-% 4-Hydroxyacetophenon, bezogen auf das Gesamtgewicht der Zubereitung, enthält.

4. Verwendung von 4-Hydroxyacetophenon in UV-Lichtschutzfilter enthaltenden kosmetischen Zubereitungen zur Reduzierung der durch die Zubereitung hervorgerufende Textilverfleckung, wobei die kosmetische Zubereitung 4-(tert-Butyl)-4'--methoxydibenzoylmethan (INCI Butyl Methoxydibenzoylmethane) und/oder 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Trieazone) enthält und die kosmetische Zubereitung bzw. das Kosmetikum von 0,01 bis 2 Gewichts-% 4-Hydroxyacetophenon, bezogen auf das Gesamtgewicht der Zubereitung, enthält.

5. Verfahren oder Verwendung nach einem der vorhergehenden Ansprüche, **dadurch gekennzeichnet, dass** die Zubereitung eine oder mehrere Komplexibildner gewählt aus der Gruppe
- 1 -Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EOTMP
- Phosphonobutan-tricarbonsäure/ PBTC
- Iminodisuccinat (IDS)
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
und/oder deren Alkalisalze enthalten.

6. Verfahren oder Verwendung nach einem der vorhergehenden Ansprüche, **dadurch gekennzeichnet, dass** das Kosmetikum 1-Hydroxy-4-methyl-6-(2,4,4-trimethylpentyl)-2(1H)-pyridon (Pirocton) und/oder dessen Monoethanolamin-Salz enthält.

7. Verfahren oder Verwendung nach einem der vorhergehenden Ansprüche, **dadurch gekennzeichnet, dass** man dem Kosmetikum Siloxanelastomere zusetzt.

8. Verfahren oder Verwendung nach einem der vorhergehenden Ansprüche, **dadurch gekennzeichnet, dass** man dem Kosmetikum Polysaccharide zusetzt.

## Claims

1. Process for facilitating the ability of cosmetic preparations comprising UV light protection filters to be washed out of textiles, wherein the cosmetic preparation comprises 4-(tert-butyl)-4'-methoxydibenzoylmethane (INCI Butyl Methoxydibenzoylmethane) and/or 2,4-bis-{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine), **characterized in that** 0.01 to 2% by weight 4-hydroxyacetophenone is added to the cosmetic, based on the total weight of the preparation.

2. Process for reducing textile staining caused by cosmetic preparations comprising UV light protection filters, wherein the cosmetic preparation comprises 4-(tert-butyl)-4'-methoxydibenzoylmethane (INCI Butyl Methoxydibenzoylmethane) and/or 2,4-bis-{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine), **characterized in that** 0.01 to 2% by weight 4-hydroxyacetophenone is added to the cosmetic, based on the total weight of the preparation.

3. Use of 4-hydroxyacetophenone in cosmetic preparations comprising UV light protection filters for facilitating the ability of the UV light protection filters to be washed out from textiles contaminated with the preparations, wherein the cosmetic preparation comprises 4-(tert-butyl)-4'-methoxydibenzoylmethane (INCI Butyl Methoxydibenzoylmethane) and/or 2,4-bis-{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine) and the cosmetic preparation or the cosmetic comprises from 0.01 to 2% by weight 4-hydroxyacetophenone, based on the total weight of the preparation.

4. Use of 4-hydroxyacetophenone in cosmetic preparations comprising light protection filters for reducing textile staining caused by the preparation, wherein the cosmetic preparation comprises 4-(tert-butyl)-4'-methoxydibenzoylmethane (INCI Butyl Methoxydibenzoylmethane) and/or 2,4-bis-{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine) and the cosmetic preparation or the cosmetic comprises from 0.01 to 2% by weight 4-hydroxyacetophenone, based on the total weight of the preparation.

5. Process or use according to any of the preceding claims, **characterized in that** the preparation comprises one or more chelating agents selected from the group comprising
- 1-hydroxyethane-(1,1-diphosphonic acid)/HEDP
- aminotrimethylenephosphonic acid/ATMP
- diethylenetriaminepenta(methylenephosphonic acid)/DTPMP
- ethylenediaminetetra(methylenephosphonic acid)/EDTMP
- phosphonobutanetricarboxylic acid/PBTC
- iminodisuccinate (IDS)
- sodium polyphosphate
- tetrasodium pyrophosphate
- hydroxamic acid
- polygalacturonic acid
- succinic acid
- formic acid
- malic acid
- ethylenediaminetetraacetic acid (EDTA)
and/or alkali metal salts thereof.

6. Process or use according to any of the preceding claims, **characterized in that** the cosmetic comprises 1-hydroxy-4-methyl-6-(2,4,4-trimethylpentyl)-2(1H)-pyridone (piroctone) and/or the monoethanolamine salt thereof.

7. Process or use according to any of the preceding claims, **characterized in that** siloxane elastomers are added to the cosmetic.

8. Process or use according to any of the preceding claims, **characterized in that** polysaccharides are added to the cosmetic.

## Revendications

1. Procédé pour la facilitation de la lessivabilité de préparations cosmétiques contenant des filtres protecteurs anti-UV, la préparation cosmétique contenant du 4-(tert.-butyl)-4'-méthoxydibenzoylméthane (INCI Butyl Methoxydibenzoylmethane) et/ou de la 2,4-bis-{[4-(2-éthyl-hexyloxy)-2-hydroxy]-phényl}-6-(4-méthoxyphényl)-1,3,5-triazine (INCI Bis-Ethylhexyloxyphenol Methoxyphenyl Triazine), **caractérisé en ce que** 0,01 à 2 % en poids de 4-hydroxyacétophénone sont ajoutés au produit cosmétique, par rapport au poids total de la préparation.

2. Procédé pour la réduction des taches sur un textile provoquées par des préparations cosmétiques contenant des filtres protecteurs anti-UV, la préparation cosmétique contenant du 4-(tert.-butyl)-4'-méthoxydibenzoylméthane (INCI Butyl Methoxydibenzoylmethane) et/ou de la 2,4-bis-{[4-(2-éthyl-hexyloxy)-2-hydroxy]-phényl}-6-(4-méthoxyphényl)-1,3,5-triazine (INCI Bis-Ethylhexyloxyphenol Methoxyphenyl Triazine), **caractérisé en ce que** 0,01 à 2 % en poids de 4-hydroxyacétophénone sont ajoutés au produit cosmétique, par rapport au poids total de la préparation.

3. Utilisation de 4-hydroxyacétophénone dans des préparations cosmétiques contenant des filtres protecteurs anti-UV pour la facilitation de la lessivabilité des filtres protecteurs anti-UV de textiles contaminés avec les préparations, la préparation cosmétique contenant du 4-(tert.-butyl)-4'-méthoxydibenzoylméthane (INCI Butyl Methoxydibenzoylmethane) et/ou de la 2,4-bis-{[4-(2-éthyl-hexyloxy)-2-hydroxy]-phényl}-6-(4-méthoxyphényl)-1,3,5-triazine (INCI Bis-Ethylhexyloxyphenol Methoxyphenyl Triazine) et la préparation cosmétique ou le produit cosmétique contenant de 0,01 à 2 % en poids de 4-hydroxyacétophénone, par rapport au poids total de la préparation.

4. Utilisation de 4-hydroxyacétophénone dans des préparations cosmétiques contenant des filtres protecteurs anti-UV pour la réduction des taches sur un textile provoquées par la préparation, la préparation cosmétique contenant du 4-(tert.-butyl)-4'-méthoxydibenzoylméthane (INCI Butyl Methoxydibenzoylmethane) et/ou de la 2,4-bis-{[4-(2-éthyl-hexyloxy)-2-hydroxy]-phényl}-6-(4-méthoxyphényl)-1,3,5-triazine (INCI Bis-Ethylhexyloxyphenol Methoxyphenyl Triazine) et la préparation cosmétique ou le produit cosmétique contenant de 0,01 à 2 % en poids de 4-hydroxyacétophénone, par rapport au poids total de la préparation.

5. Procédé ou utilisation selon l'une quelconque des revendications précédentes, caractérisé(e) en ce que la préparation contient un ou plusieurs agents de formation de complexe choisis dans le groupe
- acide 1-hydroxyéthane-(1,1-diphosphonique)/HEDP
- acide aminotriméthylènephosphonique/ATMP
- acide diéthylènetriaminepenta(méthylènephosphonique)/DTPMP
- acide éthylènediaminetétra(méthylènephosphonique)/ EDTMP
- acide phosphonobutane-tricarboxylique/PBTC
- iminodisuccinate (IDS)
- polyphosphate de sodium
- pyrophosphate tétrasodique
- acide hydroxamique
- acide polygalacturonique
- acide succinique
- acide formique
- acide malique
- acide éthylènediaminetétraacétique (EDTA)
et/ou leurs sels d'alcali.

6. Procédé ou utilisation selon l'une quelconque des revendications précédentes, caractérisé(e) en ce que le produit cosmétique contient de la 1-hydroxy-4-méthyl-6-(2,4,4-triméthylpentyl)-2(1H)-pyridone (piroctone) et/ou son sel de monoéthanolamine.

7. Procédé ou utilisation selon l'une quelconque des revendications précédentes, caractérisé(e) en ce qu'on ajoute des élastomères de siloxane au produit cosmétique.

8. Procédé ou utilisation selon l'une quelconque des revendications précédentes, caractérisé(e) en ce qu'on ajoute des polysaccharides au produit cosmétique.
